(19) Europäisches Patentamt

European Patent Office

Office européen des brevets

(11) **EP 0 944 572 B1**

(12) **EUROPEAN PATENT SPECIFICATION**

(45) Date of publication and mention
of the grant of the patent:
**10.04.2002 Bulletin 2002/15**

(51) Int Cl.⁷: **C07C 67/08**, C07C 69/14,
C07C 69/24, C07C 69/54,
C07C 67/03

(21) Application number: **97949048.9**

(22) Date of filing: **12.12.1997**

(86) International application number:
**PCT/GB97/03436**

(87) International publication number:
**WO 98/25876 (18.06.1998 Gazette 1998/24)**

(54) **PRODUCTION OF ORGANIC CARBOXYLIC ACID ESTERS**

HERSTELLUNG VON ORGANISCHEN CARBONSÄUREESTERN

PRODUCTION D'ESTERS D'ACIDES ORGANIQUES CARBOXYLIQUES

(84) Designated Contracting States:
**AT BE DE ES FR GB IT NL**

(30) Priority: **12.12.1996 ZA 9610472**

(43) Date of publication of application:
**29.09.1999 Bulletin 1999/39**

(73) Proprietor: **Sasol Technology (Proprietary)
Limited
2196 Johannesburg, Transvaal (ZA)**

(72) Inventors:
• **YOUNG, Desmond, Austin
Vanderbijlpark 1911 (ZA)**
• **STEYNBERG, Jan, Petrus
SW5 Vanderbijlpark 1911 (ZA)**
• **RADEMAN, Peter, Icobus
Vanderbijlpark 1911 (ZA)**
• **VERMAAK, Ester, Johanna
Sasolburg 9570 (ZA)**
• **SUMANI, Oliver, Lebohang, Mmontshi
Sasolburg 9570 (ZA)**
• **JAGER, Geert, Guido, Jaap
Sasolburg 9570 (ZA)**

(74) Representative: **Kador, Ulrich, Dr. et al
Kador & Partner
Corneliusstrasse 15
80469 München (DE)**

(56) References cited:
**EP-A- 0 446 106          EP-A- 0 640 583
US-A- 2 947 779          US-A- 4 018 816**

• **PATENT ABSTRACTS OF JAPAN vol. 013, no. 238 (C-603), 5 June 1989 & JP 01 050843 A (MITSUBISHI KASEI CORP), 27 February 1989,**
• **PATENT ABSTRACTS OF JAPAN vol. 006, no. 186 (C-126), 22 September 1982 & JP 57 099556 A (AGENCY OF IND SCIENCE & TECHNOL), 21 June 1982,**
• **PATENT ABSTRACTS OF JAPAN vol. 003, no. 104 (C-057), 4 September 1979 & JP 54 081233 A (UBE IND LTD), 28 June 1979,**
• **PATENT ABSTRACTS OF JAPAN vol. 001, no. 131 (C-029), 28 October 1977 & JP 52 083415 A (MITSUBISHI CHEM IND LTD), 12 July 1977,**
• **DATABASE WPI Week 9705 Derwent Publications Ltd., London, GB; AN 97-050223 XP002054681 ALIEV, A M ET AL.: "Benzyl acetate prepn." & RU 2 059 606 C (AS AZERB CHEM TECHN THEORET PROBLEMS) , 10 May 1996**

**Description**

THIS INVENTION relates to the production of organic carboxylic acid esters. It relates in particular to a process for producing carboxylic acid esters, and to a process for transesterifying an ester.

The Applicant is aware of EP-A-446106 which discloses that esterification reactions can be conducted in the gas phase in the presence of solid acid catalysts. It also discloses the use of ion exchange resins in liquid phase processes. However, it does not disclose a process for producing a carboxylic acid ester in accordance with the present invention.

According to a first aspect of the invention, there is provided a process for producing a carboxylic acid ester, which process comprises reacting a first reactant comprising at least one carboxylic acid with a second reactant comprising at least one alcohol, with the carboxylic acid and/or the alcohol being Fischer-Tropsch derived, and with the reaction being effected at elevated temperature and at a pressure such that the reactants are in a vapour phase, the reaction further being effected in the presence of a solid acid catalyst, thereby to produce at least one carboxylic acid ester.

By 'Fischer-Tropsch derived' in relation to the alcohol or carboxylic acid is meant an alcohol or carboxylic acid obtained by subjecting a synthesis gas comprising mainly carbon monoxide and hydrogen to Fischer-Tropsch synthesis reaction at elevated temperature and at elevated pressure in the presence of a Fischer-Tropsch catalyst, which is normally iron and/or cobalt based.

The solid acid catalyst is thus temperature stable at the temperature at which the reaction is effected, ie it does not deteriorate to an appreciable extent at the temperature at which the reaction is effected. The solid acid catalyst may, in particular, comprise a cationic ion exchange resin.

While, at least in principle, any suitable solid cationic exchange resin can be used as the catalyst, the catalyst may, in particular, be a sulphonic acid ion exchange resin. Such resins display good thermal stability at elevated temperature. Thus, in one embodiment of the invention, the catalyst may comprise a sulfonated polystyrene or polydivinylbenzene resin, which is typically available in the form of beads. These resins generally exhibit good thermal stability up to about 150°C, and when used as the catalyst in the process, the elevated temperature at which the reaction takes place must thus not exceed about 150°C. Examples of such sulfonated polystyrene resins are those available under the trade mark Amberlyst from Rhom and Haas France SA of La Tour de Lyon, 185 Rue de Bercy, 15579, Paris Cedex 12, France. In another embodiment of the invention, the catalyst may comprise a sulfonated polysiloxane resin. These resins generally exhibit good thermal stability up to about 230°C, and when used as the catalyst in the process, the elevated temperature at which the reaction takes place must thus not exceed about 230°C. An example of such a sulfonated polysiloxane resin is that available under the trade mark Deloxan ASP from Degussa AG of Weissfrauenstrasse 9, D-60287, Frankfurt/Main, Federal Republic of Germany. In yet another embodiment of the invention, the catalyst may comprise a sulfonated perfluorinated resin, eg a sulfonated poly-perfluoroethylene resin. These resins generally exhibit good thermal stability up to about 200°C, and when used as the catalyst in the process, the elevated temperature at which the reaction takes place must thus not exceed 200°C. An example of such a sulfonated perfluorinated resin is that available under the trade mark Nafion from Du Pont Nafion of PO Box 80323, Wilmington, DE, USA, 19880-0323.

The elevated temperature at which the reaction is effected must thus, as indicated above, be below the upper temperature limit of the catalyst, ie must be at a temperature at which the catalyst is still stable. The Applicant has found that when a carboxylic acid/alcohol binary mixture is used as a feedstock to a reaction zone in which the reaction takes place, the fact that such a binary mixture normally has a boiling or vaporization point lower than the boiling point of the highest boiling point component of the mixture, permits the vapour or gas phase reaction to be effected at a temperature below the upper temperature limit of the catalyst. Thus, for example, the binary feedstock mixture can be selected to vaporize at a temperature below 150°C, eg at about 105°C when using a binary mixture suitable for the vapour phase production of ethyl acetate, so that the esterification reaction can thus be effected at this temperature, when the sulfonated polystyrene or divinylbenzene resin is used as the catalyst.

The fact that a binary mixture of a carboxylic acid and an alcohol normally has a boiling or vaporization point lower than the boiling point of the highest boiling component of the mixture, permits the vapour phase esterification of the process to be effected at the lowest possible temperature. Additionally, as indicated hereinbefore, it also permits relatively high boiling components, ie boiling at about or above the upper temperature limit of a particular catalyst, to be used. Thus, for example, when a sulfonated polystyrene or polydivinylbenzene resin is used as the catalyst, a Fischer-Tropsch derived acetic acid/propionic acid binary mixture can be used, even though propionic acid has a boiling point (at atmospheric pressure) of about 141°C, ie at about the upper temperature limit of 150°C of the resin, since the boiling point (at atmospheric pressure) of a typical Fischer-Tropsch derived binary mixture consisting of about 69,8% acetic acid and 30,2% propionic acid is only about 121°C.

The reaction of the first and second reactants may thus be effected in a reaction zone containing a fixed bed of the catalyst in particulate form, eg bead form. The reactants pass through the bed, with the reactants being fed into

EP 0 944 572 B1

the reaction zone as a vaporized feedstock comprising said binary mixture of the reactants. The vaporized feedstock typically passes through the catalyst bed on a continuous basis, and in an upward or downward direction. Product, ie carboxylic acid ester(s), will then also be withdrawn continuously from the reaction zone.

The pressure at which the reaction is effected will thus be dependent on the carboxylic acid and the alcohol used, as well as on the reaction temperature, and will naturally be selected such that the reactants are in the vapour phase. The pressure can thus vary widely, from a sub-atmospheric pressure to a pressure above atmospheric pressure, eg from about 0,05 bar(a) (5 kPa(a)) to about 3 bar(a) (300 kPa(a)). Thus, any carboxylic acid/alcohol binary mixture that vaporizes at a temperature below the upper limit of the catalyst that is used, eg below 150°C when a sulfonated polystyrene resin is used as the catalyst, can be esterified at atmospheric pressure provided that, as mentioned hereinbefore, the carboxylic acid and/or the alcohol is Fischer-Tropsch derived. A carboxylic acid/alcohol binary mixture that vaporizes at higher temperatures at atmospheric pressure can also be used, by effecting the esterification at a sub-atmospheric pressure at which the binary mixture is in vaporized form.

As hereinbefore described, at least one of the carboxylic acid and the alcohol are Fischer-Tropsch derived. Thus, when the carboxylic acid is Fischer-Tropsch derived, the alcohol may be non-Fischer-Tropsch derived, and vice versa. However, both the carboxylic acid and the alcohol may be Fischer-Tropsch derived.

The second reactant may comprise Fischer-Tropsch derived methanol, ethanol, propanol, i-propanol, butanol, 2°-butanol, i-butanol or mixtures of two or more such alcohols. In particular, the second reactant may comprise a Fischer-Tropsch derived alcohol mixture. Examples of such Fischer-Tropsch derived alcohol mixtures are ethylol which typically comprises, on a mass basis, 94,6% ethanol, 4,8% i-propanol, and 0,6% other alcohols; propylol which typically comprises, on a mass basis, 87,8% propanol, 11,0% 2°-butanol, 1,0% i-butanol, and 0,2% other alcohols, and sabutol which typically comprises, on a mass basis, 64,3% butanol, 16,06% 2-pentanol, 11,91% i-butanol, 4,03% 3-methyl-2-butanol, and 3,7% other alcohols. The compositions of these Fischer-Tropsch derived alcohol mixtures can, however, vary in practice. With alcohol feeds such as these, the pressure at which the reaction is effected will typically be sub-atmospheric pressure or greater.

Higher boiling point alcohols can, however, also, as hereinbefore described, be esterified in accordance with the present invention by vaporizing said alcohols under reduced pressure, ie at sub-atmospheric pressure, at temperatures that fall within the stability range of cationic ion exchange resin catalysts. These alcohols may include Fischer-Tropsch derived pentanol, hexanol, heptanol, ethylene glycol, propylene glycol, phenol, cresol, or, in principle, any other alcohol that can be vaporized below 150°C, 200°C or 230°C for a sulfonated polystyrene or polydivinylbenzene resin, a sulfonated perfluorinated resin and a sulfonated polysiloxane resin, respectively, as the catalyst, at atmospheric or sub-atmospheric pressure.

The first reactant may comprise acetic acid, propionic acid, acrylic acid, methacrylic acid, or mixtures of two or more thereof, which may, as hereinbefore described, be Fischer-Tropsch derived. In particular, the first reactant may comprise a Fischer-Tropsch derived carboxylic acid or carboxylic acid mixture. Examples of such Fischer-Tropsch derived carboxylic acids and carboxylic acid mixtures include acetic acid, propionic acid and acetic acid/propionic acid mixtures.

Higher boiling point carboxylic acids can also be esterified in accordance with the present invention by vaporizing said acids under reduced pressure, ie at sub-atmospheric pressure, at temperatures that fall within the stability range of cationic ion exchange resin catalysts. These acids include butyric, pentanoic, hexanoic acid, or, in principle, any other acid that can be vaporized below 150°C, 200°C or 230°C depending on the resin used as the catalyst, at atmospheric or sub-atmospheric pressure.

In principle, any combination of alcohols and organic or carboxylic acids that can be vaporized at reduced or sub-atmospheric pressures at temperatures below 150°C, 200°C or 230°C, depending on the resin used as catalyst, can thus be esterified in the fixed bed cationic resin process of the invention.

The molar ratio of carboxylic acid(s) to alcohol(s) may vary widely, typically between about 8,0:0,5 to about 0,5:8,0, eg between about 4:1 to about 1:4; however, it is believed that a molar excess of carboxylic acid(s) over alcohol (s) will be beneficial, eg enhance catalyst stability over a period of time, as well as eliminate or at least reduce unwanted etherication and dehydration (secondary and tertiary alcohols) side reactions. Thus, for example, the molar ratio of carboxylic acid(s) to alcohol(s) can typically be about 1,5:1,0.

The vaporized feedstock may include up to about 8% by mass of vaporized water, based on the total feedstock. The vaporized feedstock may also include up to about 20% by mass of the carboxylic acid ester(s) produced in the process, as a recycle component.

The Applicant has unexpectedly found that, by using the combination of the vapour phase reaction and the cationic exchange resin catalyst, high conversions and high selectivities or yields, at high throughputs, are obtainable with Fischer-Tropsch derived reactants. Thus, conversions of at least 93%, typically 96% or higher, are achievable, at a product yield or selectivity between 83% and 99%, typically about 95%, and at a liquid hourly space velocity between 0,1 and 20,0, typically between 2,5 and 5,0. In other words, when regard is had to the esterification reaction which takes place, ie

$$R_1COOH + R_2OH \Leftrightarrow R_1COOR_2 + H_2O \qquad (1)$$

where $R_1$ and $R_2$ are hydrocarbon radicals, the esterification reaction equilibrium is shifted significantly to the right hand side of reaction (1) in the present invention, and this is achieved without having to remove water.

It is known to those skilled in the art that the equilibrium constant K, where K= $[R_1COOR_2] \cdot [H_2O] /[R_1COOH] \cdot [R_2OH]$, has a value of 4 for a liquid phase sulphuric acid catalyzed ethyl acetate esterification process when equimolar quantities of ethanol and acetic acid are used as reactants. In the process of the present invention, the equilibrium constant has unexpectedly been found to have a value of 21-26 when acetic acid and ethanol are used in a 1:1 molar ratio as vaporized feedstock. The relevance of this significant shift of the equilibrium towards the ester product is even greater if it is taken into account that contamination of the feedstock with up to 8% (by mass) water (based on the total feedstock mass) has no adverse effect on the conversion to the ester product.

Based on known liquid phase esterification technology, it would have been expected that higher reaction temperatures would increase reaction kinetics, but would also simultaneously result in increased unwanted side reactions. However, as indicated hereinbefore, it was surprisingly found that in the process of the present invention, high conversions and high selectivities are obtained using Fischer-Tropsch derived reactants, with a low level of unwanted side reactions, ie decrease selectivity. This unexpectedly permits the use of a relatively simple process, as regards product work-up, when using Fischer-Tropsch derived feedstocks, since the high conversions and high selectivities of the process render separation of unreacted reactants and unwanted side effects relatively straight-forward. This was unexpected in view of the range of components present in Fischer-Tropsch streams, eg Fischer-Tropsch alcohol streams.

In other words, it was surprisingly found that the reaction between the chemisorbed carboxylic acid and alcohol is profoundly faster than the reaction between the chemisorbed ester and water that results in hydrolysis of the ester product as well as faster than the reaction between two alcohol molecules that results in unwanted dehydration to ether and olefin side products, when the esterification reaction is performed over a resin catalyst in the vapour phase, particularly when the carboxylic acid is an excess reagent. This not only results in a significant shift of the esterification equilibrium towards formation of the ester product, to obtain conversions in excess of 93% as hereinbefore described, but furthermore allows carboxylic acids and alcohols to be esterified with excellent selectivity, typically having a yield in excess of 95%, as also hereinbefore described.

Due to the complexity of Fischer-Tropsch product streams, it is normally uneconomic to purify the alcohols obtained from the Fischer-Tropsch process to a purity in excess of 99%. Fischer-Tropsch derived alcohols are therefore generally contaminated with other alcohols that are often of the secondary type. Fischer-Tropsch derived ethylol, for example, can also typically consist of 94,8% ethanol, 4,39% isopropanol and 0,81% other impurities. Since 2° alcohols dehydrate to olefins with greater ease than 1° alcohols, selectivity towards the ester product is of utmost importance if Fischer-Tropsch derived alcohol streams are to be esterified using resin catalysis, without the resin catalyst being fouled by olefin oligomers. It was thus surprisingly found that the process of the present invention is particularly suitable for esterifying carboxylic acids with Fischer-Tropsch derived alcohols.

The Applicant has also surprisingly found that transesterification of an ester with a Fischer-Tropsch derived alcohol can be effected by similarly reacting the ester with the alcohol at elevated temperature and at a pressure such that the reactants are in a vapour phase, with the reaction being effected in the presence of a temperature stable cationic ion exchange resin as a solid catalyst. The reaction parameters and catalyst can thus be as hereinbefore described.

The ester can thus be that obtained by reacting a carboxylic acid with an alcohol, as hereinbefore described. Instead, however, the ester can be that derived from another source.

Thus, according to a second aspect of the invention, there is provided a process for transesterifying an ester, which process comprises reacting a first reactant comprising an ester, with a second reactant comprising a Fischer-Tropsch derived alcohol, at elevated temperature and at a pressure such that the reactants are in a vapour phase, the reaction being effected in the presence of a solid acid catalyst.

As stated hereinbefore, the reaction parameters and catalyst can be as hereinbefore described.

In one embodiment of the invention, the ester may be ethyl acetate. The alcohol may then be propanol, 2°-propanol, butanol, 2°-butanol, i-butanol, or mixture of two or more such alcohols. The pressure may then be sub-atmospheric pressure or greater.

The invention will now be described by way of example with reference to the accompanying diagrammatic drawings and to the non-limiting examples set out hereinafter.

In the drawings,

FIGURE 1 shows a simplified flow diagram of a process according to a first embodiment of the invention, for producing carboxylic acid esters; and

FIGURE 2 shows a simplified flow diagram of a process according to a second embodiment of the invention, for producing carboxylic acid esters.

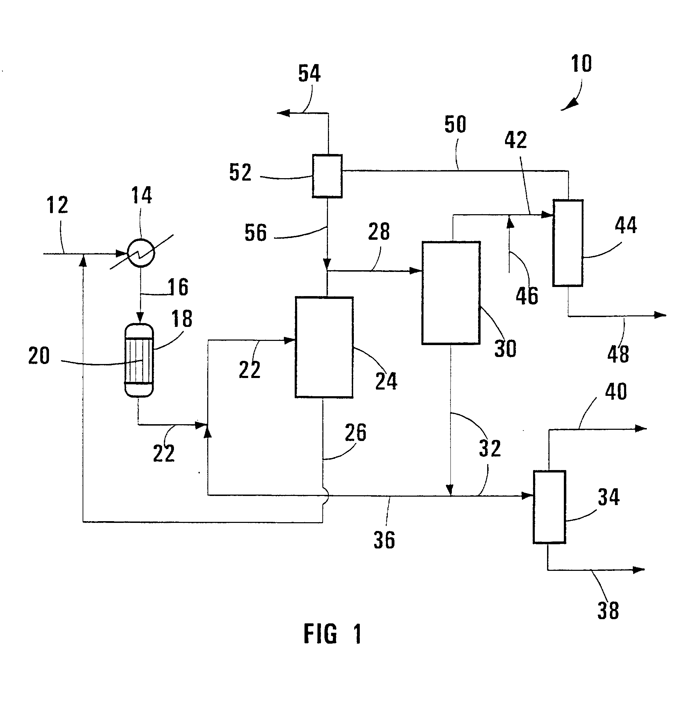

Referring to Figure 1, reference numeral 10 generally indicates a process according to a first embodiment of the invention, for producing carboxylic acid esters.

The process 10 comprises a feed line 12 leading to a feed vaporizer 14. A vaporized feed line 16 leads from the vaporizer 14 to an esterification reactor 18 providing an esterification reaction zone. The reactor 18 contains a catalyst bed 20 of solid catalyst particles comprising an ion exchange resin containing sulphonic acid groups.

A flow line 22 leads from the reactor 18 to an acid recovery column 24. A recycle acetic acid line 26 leads from the bottom of the column 24 to the flow line 12. A wet product withdrawal line 28 leads from the top of the column 24 to an acetate drying column 30. A bottoms withdrawal line 32 leads from the bottom of the column 30 to an ethyl acetate recovery column 34, while an ethyl acetate recycle line 36 leads from the line 32 to the line 22. A bottoms product withdrawal line 38 leads from the bottom of the column 34, while an ethyl acetate overheads line 40 leads from the top of the column 34.

An overheads withdrawal line 42 leads from the top of the column 30 to a water/ethanol removal column 44. A water make-up line 46 leads into the line 42. An effluent line 48 leads from the bottom of the column 44, while an ethyl acetate azeotrope line 50 leads from the top of the column 44 to a flash separation drum 52. An ether withdrawal line 54 leads from the drum 52 to flare. An ethyl acetate recycle line 56 leads from the drum 52 to the flow line 28.

In use, a feedstock comprising a Fischer-Tropsch derived ethyl alcohol/isopropyl alcohol mixture containing some water, typically not exceeding 8% by mass based on the total feedstock, and a molar excess of acetic acid, enters the process 10 along the flow line 12. This feed stream is admixed, by means of the flow line 26, with recycled acetic acid, to produce a composite feed stream. The composite feed stream is completely vaporized in the vaporizer 14 by heating it to a temperature up to 230°C (depending on the resin used as catalyst in the reactor 18), eg about 105°C. The vaporized composite feed passes along the line 16 to the reactor 18. The reactor 18 operates at a pressure of about 1 bar(a) (100 kPa(a)).

In the reactor 18, part of the acid/alcohol mixture in the composite feed is esterified by reaction catalyzed by the catalyst bed 20, to yield a corresponding mixture of esters. A product stream, comprising the corresponding esters, unreacted acetic acid, water produced by esterification, and unreacted ethanol, passes along the flow line 22, to the acid recovery column 24. The column operates at about 1 bar(a) (100 kPa(a)). Recycled recovered ethyl acetate mixture, containing some isopropyl acetate, from the column 30 is admixed with the feed to column 24, to aid in azeotropic removal of water from unreacted acetic acid destined for recycle back to the reactor 18 via the recycle line 26.

Wet ethyl acetate vapour mixture is recovered as an overhead product in the column 24 and passes along the flow line 28 into the acetate drying column 30. The column 30 operates at a pressure of about 8 bar(a) (800 kPa (a)). Most of the unreacted ethanol and water formed by reaction in the reactor 18, together with the corresponding azeotropic amount of ethyl acetate, is recovered as an overhead fraction in the column 30, and exits the column 30 along the flow line 42.

Dry ethyl acetate/isopropyl acetate mixture is recovered as a bottoms fraction in the column 30, and withdrawn along the line 32. Part of this mixture is recycled back to the column 24 along the flow line 36, for azeotropic removal of water from acid as hereinbefore described. The balance of the mixture enters the column 34, where pure, eg 99,8% by mass, ethyl acetate is recovered as an overhead fraction, and withdrawn along the flow line 40. A mixture of mostly isopropyl acetate and ethyl acetate is removed from the column 34 as a bottoms fraction, along line 38. Column 34 operates at a pressure of about 1 bar(a) (100 kPa(a)).

Water is admixed with the overheads from the column 30, the water entering along the flow line 46. The water aids with the removal of unreacted ethanol and water from the ethyl acetate, in the column 44. Water and ethanol is withdrawn from the column 44 along line 48 as an effluent, while ethyl acetate is recovered, together with azeotropic water and ethanol, along the flow line 50. The flow line 50 leads to the flash drum 52 which operates at a pressure of about 1 bar(a) (100 kPa(a)). The flash drum 52 serves to purge any ethers present due to by-products formation in the reactor 18 and impurities present in the Fischer-Tropsch alcohol mixture. The ethers are withdrawn along the flow line 54.

The ether free ethyl acetate mixture is recycled back to the ethyl acetate drying columns 30, along the flow lines 56, 28.

It is to be appreciated that when a different alcohol/carboxylic acid mixture is used as a feed to the reactor 18, and/or when a different reaction temperature is used in the reactor 18, a different operating pressure, typically between 0,05 and 3 bar(a) (5 and 300 kPa(a)), can be used in the reactor 18, with the pressure being selected such that the feed mixture is in the vapour phase at the reaction temperature in question. The column 24 can then operate at a pressure to match that of the reactor 18, eg at a pressure between 0,05 and 10 bar(a) (5 kPa and 1000 kPa(a)). Similarly, the columns 30 and 34, and the flash drum 52, can then operate at a pressure to match that of the reactor 18 and the column 24, eg at a pressure between 0,05 and 10 bar(a) (5 kPa and 1000 kPa(a)).

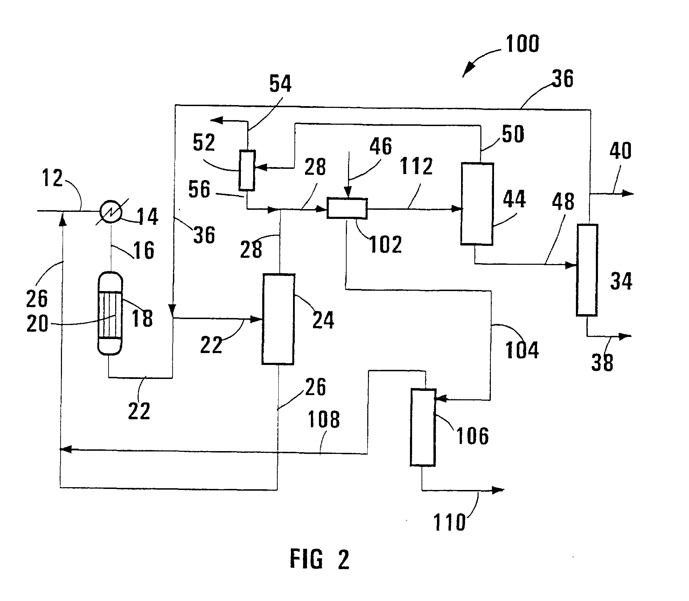

Referring to Figure 2, reference numeral 100 generally indicates a process according to a second embodiment of the invention, for producing carboxylic acid esters.

Parts of the process 100 which are the same or similar to those of the process 10 hereinbefore described with reference to Figure 1, are indicated with the same reference numerals.

The process 100 also comprises the feed line 12 leading to the feed vaporizer 14. As in Figure 1, the vaporized feed line 16 leads from the vaporizer 14 to the esterification reactor 18, with the flow line 22 leading from the reactor 18 to the acid recovery column 24. As in Figure 1, the recycle acetic acid line 26 leads from the bottom of the column 24 to the flow line 12. The wet product withdrawal line 28 from the top of the column 24 leads to a phase separator 102. A bottoms withdrawal line 104 leads from the phase separator to an alcohol/ethyl acetate recovery column 106. An overheads withdrawal line 108 from the column 106 leads to the line 26, while a water withdrawal line 110 leads from the bottom of the column 106. An overheads line 112 leads from the phase separator 102 to the water/ethanol removal or acetate recovery column 44. The water make-up line 46 leads into the phase separator 102.

The ethyl acetate or overheads line 50 from the top of the column 44 leads to the flash separation drum 52, as in Figure 1. The effluent or bottoms withdrawal line 48 from the bottom of the column 44 leads to the ethyl acetate recovery column 34, while the ethyl acetate recycle line 36 leads from the line 48 to the line 22.

In use, feedstock comprising a Fischer-Tropsch derived ethyl alcohol/isopropyl alcohol mixture containing some water, typically not exceeding 8% by mass based on the total feedstock, and a molar excess of acetic acid, enters the process along flow line 12. This feed stream is admixed, by means of flow line 26, with recycled acetic acid and an ethyl acetate/ethanol/water mixture, entering the flow line 26 along the flow line 108, to produce a composite feed stream. The composite feedstream is completely vaporized in the vaporizer 14 by heating it to a temperature up to 230°C (depending on the resin used on the reactor 20), eg to about 105°C. The vaporized composite feed passes along line 16 to the reactor 18. The reactor 18 operates at a pressure of about 1 bar(a) (100 kPa(a)).

In the reactor 18, portions of the acid and alcohol mixture in the composite feedstream are esterified by reaction catalyzed by the catalyst bed 20, to yield a corresponding mixture of esters. A product stream comprising the corresponding esters, unreacted acetic acid, water produced by the esterification reaction, and unreacted ethanol, passes along the flow line 22, to the acid recovery column 24. The column 24 operates at about 1 bar(a) (100 kPa(a)). Recovered ethyl acetate is recycled from the column 34 along the line 36, and is admixed with the feed to column 24, to aid in the azeotropic removal of water from the unreacted acetic acid destined for recycle back to the reactor 18 along line 26.

Wet ethyl acetate vapour mixture is recovered as an overhead product in the column 24 and passes along the flow line 28 into the phase separator 102. The phase separator 102 operates at a pressure of 1 bar(a) (100 kPa(a)). Water is admixed with the overheads from the column 24 in the phase separator 102, with the water entering the phase separator along flow line 46. Most of the unreacted ethanol and water formed by the reaction in reactor 18, together with the water added along flow line 46 to aid phase separation, is recovered as an aqueous phase that exits the phase separator 102 along flow line 104. The aqueous phase is saturated with ethyl acetate.

The aqueous phase enters the alcohol/ethyl acetate recovery column 106 where it is distilled. The ethanol and ethyl acetate is thus stripped from the water and exits the column 106 along flow line 108. A mixture containing unreacted ethanol, ethyl acetate and stripping water is thus recycled to the reactor 18 along flow line 26. Pure water exits the column 106 along flow line 110 as an effluent.

The organic phase, containing most of the ethyl acetate mixture, saturated with water, is recovered in the phase separator 102 and exits along flow line 112, to the acetate recovery column 44. The column 44 operates at about 1 bar(a) (100 kPa(a)). The remainder of the unreacted ethanol and water in the organic phase, together with the corresponding azeotropic amount of ethyl acetate, is recovered as an overheads fraction in the column 44, and exits the column 44 along flow line 50. It thus passes to the flash drum 58 which operates at a pressure of about 1 bar(a) (100 kPa(a)). The flash drum serves to purge any ethers present due to by-products formation in the reactor 18 and impurities in the Fischer-Tropsch alcohol mixture. The ethers are withdrawn along the flow line 54.

The ethyl acetate mixture is recycled back to the phase separator 102, along flow lines 56, 28.

Dry ethyl acetate/isopropyl acetate is recovered as the bottoms fraction in the acetate recovery column 44, and withdrawn along the line 48. This mixture enters the column 34, where pure, eg 99,8% by mass, ethyl acetate is recovered as an overhead fraction, and withdrawn along flow line 40. Part of this ethyl acetate is recycled back to the column 24 along flow line 36 for the azeotropic removal of water from the acid as hereinbefore described. A mixture of mostly isopropyl acetate and ethyl acetate is removed from the column 34 as a bottoms fraction, along line 38. Column 34 operates at a pressure of 1 bar(a) (100 kPa(a)).

The feasibility of the processes 10, 100 were verified on laboratory scale, in the following non-limiting examples.

<u>EXAMPLE 1</u>

This example illustrates, in accordance with the invention, vapour phase esterification of acetic acid and ethylol to produce ethyl acetate. A reactor feed consisting of acetic acid (99,50%) and ethanol (94,63%) was used. The alcohol component or second reactant also contained 4,77% isopropanol and 0,60 % of other impurities, which is typical of a Fischer-Tropsch ethylol product. The acetic acid and ethanol were mixed in a 1,62:1,0 molar ratio. The feed was vaporized at 105°C, and fed continuously into the bottom of a stainless steel reactor (25mm diameter) packed with Amberlyst 36 sulphonic acid ion exchange resin beads as catalyst, and which were preheated to 105°C. The reactor was operated continuously at a LHSV of 2,5 for 10 days. Reactor effluent was condensed at 0°C in a cooler and analyzed gas chromatographically. Table 1 depicts the conversion of ethanol and yield of ethyl acetate respectively over time.

Conversion and yield were calculated as follows:

conversion of ethanol (%) = {(moles of ethanol fed -

moles of ethanol unreacted)

/ moles of ethanol fed} x

100

yield of ethyl acetate (%) = {moles of ethyl acetate

formed / moles of ethanol

fed} x 100

TABLE 1

| Elapsed reaction time | Conversion of ethanol % | Yield of ethyl acetate % |
|---|---|---|
| 1 day | 94,20 | 95,72 |
| 2 days | 93,90 | 95,60 |
| 4 days | 94,10 | 95,73 |
| 6 days | 93,60 | 95,61 |
| 8 days | 93,70 | 95,64 |
| 10 days | 93,53 | 95,63 |

It can be seen that there was no significant deterioration in either conversion or yield over the 10 days at the test, thus indicating good catalyst stability.

<u>EXAMPLE 2</u>

This example illustrates the conversions and yields recorded for the esterification of acetic acid with various alcohols. The reactor was operated at a LHSV of 5. Except for a different feed composition (feed always consisted of the alcohol and acetic acid in a 1,5:1,0 molar ratio) the same operating conditions and setup as described in Example 1 were used to obtain the results summarised in Table 2.

TABLE 2

| Alcohol used | Conversion of acetic acid % | Yield of acetate % |
|---|---|---|
| Methanol | 94,31 | 99,00 |
| Ethanol | 91,47 | 89,24 |

TABLE 2 (continued)

| Alcohol used | Conversion of acetic acid % | Yield of acetate % |
|---|---|---|
| Propanol | 92,89 | 89,39 |
| 2°-propanol | 79,10 | 75,36 |
| Butanol | 93,86 | 92,97 |
| 2°-butanol | 81,67 | 71,20 |

EXAMPLE 3

This example illustrates, in accordance with the invention, vapour phase esterification of acetic acid and sabutol, a typical Fischer-Tropsch derived $C_4$ alcohol blend, to produce sabutol acetates over Deloxan ASP sulfonated polysiloxane catalyst. A reactor feed consisting of acetic acid (99,50%) and sabutol (64,3% n-butanol) was used. The alcohol component or second reactant also contained 16,06% of a $C_5$ alcohol isomer not yet unambiguously identified (2-pentanol or 3-methyl-2-butanol) as well as 15,94% of mostly secondary $C_4$ alcohol isomers (probably 3-butanol, and isobutanol) and 3,7% other alcohols by mass. Except for different feed composition (the feed always consisted of the alcohol and carboxylic acid in a 1:2 molar ratio) the same operating conditions and setup as described in Example 1 were used to obtain the results summarized in Table 3.

TABLE 3

| Component | % Conversion |
|---|---|
| n-butanol | 97 |

EXAMPLE 4

This example illustrates the conversions recorded for the esterification of various higher boiling carboxylic acids and alcohols under reduced pressures over a solid acid cation exchange resin catalyst stable up to 150°C, eg sulfonated poly-(divinylbenzene)/styrene type.
Except for different feed composition, pressure and temperature (the feed always consisted of the alcohol and carboxylic acid in a 1:2 molar ratio) the same operating conditions, catalyst and setup as described in Example 1 were used to obtain the results summarized in Table 4.

In Table 4, selectivity (%) = {moles of ester / (moles of

ester + moles of by-products)} x 100

TABLE 4

| Alcohol used | Carboxylic acid used | Vaporizing Temperature (°C) | Pressure (mbar) | % Conversion | % Selectivity |
|---|---|---|---|---|---|
| Propanol | Propionic | 100 | 854 | 80,3 | 83,5 |
| | | 100 | 400 | 78,4 | 84,5 |
| | | 100 | 200 | 63,1 | 83,5 |
| n-Butanol | Acrylic | 104 | 284 | 40,7 | 96,4 |
| | | 121 | 280 | 48,5 | 87,8 |
| n-Butanol | Butyric | 100 | 140 | 93,8 | 98,9 |
| | | 100 | 144 | 89,6 | 99 |

Table 5 summarize the results obtained when using a solid acid resin catalyst stable up to 230 °C, e.g. sulfonated polysiloxane type. Except for different feed composition and temperature (the feed always consisted of the alcohol and carboxylic acid in a 1:2 molar ratio) the same operating conditions and setup as described in Example 1 was used.

TABLE 5

| Alcohol used | Carboxylic acid used | Vaporizing Temperature (°C) | Pressure (mbar) | % Conversion | % Selectivity |
|---|---|---|---|---|---|
| n-Butanol | Acrylic | 130 | 854 | 46,8 | 88 |
| n-Butanol | Butyric | 150 | 854 | 94,5 | 92,9 |
| *n-Butanol | Acrylic | 130 | 854 | 43,37 | 68,88 |

* results obtained when excess alcohol was used in the feed, under the same conditions.

EXAMPLE 5

This example illustrates the conversions recorded for the esterification of n-butanol and ethanol with acetic acid using a solid acid cation exchange resin catalyst stable up to 230°C, ie Deloxan ASP sulfonated polysiloxane catalyst. Except for different feed composition and temperature (the feed always consisted of the alcohol and carboxylic acid in a 1:2 molar ratio) the same operating conditions and setup as described in Example 1 were used to obtain the results summarized in Table 6.

TABLE 6

| Alcohol used | Vaporizing Temperature (°C) | % Conversion | % Selectivity |
|---|---|---|---|
| Ethanol | 105 | 93 | 99 |
| n-Butanol | 125 | 96 | 99 |

EXAMPLE 6

This example illustrates the conversions recorded for the esterification of n-butanol with acetic acid using a solid acid cation exchange resin catalyst stable up to 230°C, ie Deloxan ASP sulfonated polysiloxane catalyst. Except for different LHSV (the feed always consisted of the alcohol and carboxylic acid in a 1:2 molar ratio) the same operating conditions and setup as described in Example 1 were used to obtain the results summarized in Table 7.

TABLE 7

| Vaporizing Temperature (°C) | LHSV | % Conversion | % Selectivity |
|---|---|---|---|
| 125 | 2,5 | 96 | 99 |
| 130 | 2,5 | 97 | 99 |
| 130 | 7,5 | 96 | 99 |
| 130 | 15 | 96 | 99 |

EXAMPLE 7

This example illustrates the conversions recorded for the esterification of ethanol with acetic acid using a solid acid cation exchange resin catalyst stable up to 150°C, eg sulfonated poly-(divinylbenzene)/styrene type, with up to 20% by mass ethyl acetate recycled back to the reactor. Except for different volumes of ethyl acetate in the feed (the feed always consisted of the alcohol and carboxylic acid in a 1:2 molar ratio) the same operating conditions, catalyst and setup as described in Example 1 were used to obtain the results summarized in Table 8.

TABLE 8

| Mass % excess ethyl acetate in feed | % Conversion |
|---|---|
| 0 | 92,3 |
| 5 | 92,3 |
| 10 | 92,3 |

TABLE 8 (continued)

| Mass % excess ethyl acetate in feed | % Conversion |
| --- | --- |
| 15 | 92 |
| 20 | 91,8 |

The work-up section of an esterification process generally deals with the manipulation of azeotropes in order to recover the ester product as well as excess reactants/reagents for recycle back to the reaction zone. In the process 10, the azeotropes are manipulated by means of pressure swing distillation ('PSD'). This approach is ideally suited for situations where water/alcohol/ester azeotropes do not give sufficient phase separation in the liquid phase.

There are, however, cases where a distillation-phase-separation approach is preferred, from an economical point of view. The process 100 is based on such an approach.

For those skilled in the art of esterification chemistry, it is evident that driving of the equilibrium reaction to completion and recovery of the ester product from the resulting alcohol/water/ester azeotropes are the major issues that need to be addressed during the development of an esterification process. Current processes typically apply a variety of distillation methods to effect ester and water removal from the esterification reaction in order to drive the reaction to completion. Esters of medium volatility generally form ternary azeotropic mixtures consisting of ester, alcohol and water thus allowing for the ester and water products to be removed in a reactive distillation column by means of azeotropic distillation. Since the composition of the ternary azeotrope is seldom such that ester and water products are removed in equal proportions, ester and water build-up in the system is a common problem associated with a reactive distillation approach that can only be solved by recycling huge volumes of alcohol back to the reactive distillation column in order to remove the surplus water or ester azeotropically. The much-simplified work-up sections of the processes 10, 100 as a direct result of a high per pass conversion to ester products, therefor represents a significant advantage of the present invention over known esterification processes.

The Applicant has found that the following further advantages arise when using the present invention:

- the lesser amount of alcohol in the reactor product, as compared to the reaction product when using known esterification processes, results in a more cost effective work-up of the product in situations where difficult to separate azeotropes are formed between the excess alcohol, reaction water and ester products, especially when Fischer-Tropsch alcohol mixtures are used as feed;
- catalyst fouling by olefinic by-products that result from alcohol dehydration, is suppressed;
- recycle of wet feed (up to 8% $H_2O$) has no adverse effect on the conversion to ester products;
- there is little or no decrease in catalyst life due to attrition as would arise if, for example, a fluidized bed were used;
- there is no necessity to remove ester or water in order to shift the equilibrium of reaction (1) to the right hand side;
- higher conversions than are obtainable with known liquid phase esterification reactions which are limited by the continuous azeotropical removal of water as well as the recycle of huge volumes of excess reagent that is needed to ensure acceptable conversion to the ester product, are achievable;
- since a solid resin is used as a heterogeneous catalyst, the product is easily separated therefrom, contamination of the product with catalyst is avoided, and effluent problems are minimized.

The Applicant has also found that the resin catalyst's performance in a vapour phase process as hereinbefore described appears to be more stable over time when an excess acid (typically 1,5:1,0 molar ratio)is used in the feed compared to situations where an excess alcohol (typically 1,5:1,0 molar ratio) is employed. Since ethanol contaminated with some isopropanol, as found in the Fischer-Tropsch ethylol mixture, was used as the alcohol in tests, such as in Example 1, the Applicant believes, without wishing to be bound by this theory, that this may arise from the fact that undesirable side reactions where the secondary alcohol is dehydrated to olefinic by-products that can foul the polymer beads, are inhibited when excess acid is used. The efficient way in which secondary alcohols, especially when in mixtures as is the case with Fischer-Tropsch alcohols, are dealt with reflects another significant advantage of an excess acid vapour phase esterification process in accordance with the invention.

Known liquid-phase esterifications where an excess alcohol is employed to shift the equilibrium towards esterification, ie to shift reaction (1) to the right hand side, is often hampered by the effort that needs to be invested in order to recycle the excess alcohol feed. This is especially true for the production of ethyl acetate from acetic acid and ethanol where skilful azeotropic distillations are needed to recover the excess ethanol for recycle. For those skilled in the art, it is clearly evident that acetate/alcohol mixtures would further complicate the recycle/azeotropic work-up required by the conventional process. Based on the principle that ethyl acetate can be used to dry acetic acid in extractive distillation, the current invention allows for a more cost effective product work-up if an excess acid approach is utilized to produce ethyl acetate. In contrast to excess alcohol processes where the recovery and drying of the excess alcohol feed is complex, acetic acid is fully recovered in a pure enough form to be recycled in a first downstream product distillation step.

Since the contact time of the reagents with the resin catalyst in the vapour phase process of the invention is short in comparison with liquid-phase esterification processes, the current invention furthermore allows for the esterification of unsaturated carboxylic acids (eg acrylic and methacrylic acid) with suppressed polymerization of the starting materials and products, as hereinbefore stated.

**Claims**

1. A process for producing a carboxylic acid ester, which process comprises reacting a first reactant comprising at least one carboxylic acid with a second reactant comprising at least one alcohol, with the carboxylic acid and/or the alcohol being Fischer-Tropsch derived, and with the reaction being effected at elevated temperature and at a pressure such that the reactants are in a vapour phase, the reaction further being effected in the presence of a cationic ion exchange resin as a temperature stable solid acid catalyst, thereby to produce at least one carboxylic acid ester.

2. A process according to Claim 1, wherein the cationic ion exchange resin is a sulfonated polystyrene or polydivinylbenzene resin, a sulfonated polysiloxane resin, or a sulfonated perfluorinated resin.

3. A process according to Claim 2, wherein the sulfonated polystyrene resin is used as the catalyst, and wherein the elevated temperature at which the reaction takes place does not exceed 150°C.

4. A process according to Claim 2, wherein the sulfonated polysiloxane resin is used as the catalyst, and wherein the elevated temperature at which the reaction takes place does not exceed 230°C.

5. A process according to Claim 2, wherein the sulfonated perfluorinated resin is used as the catalyst, and wherein the elevated temperature at which the reaction takes place does not exceed 200°C.

6. A process according to any one of Claims 1 to 5 inclusive, wherein the reaction of the first and second reactants is effected in a reaction zone containing a fixed bed of the catalyst in particulate form through which the reactants pass, with the reactants being fed into the reaction zone as a vaporized feedstock comprising a binary mixture of the reactants which has a boiling or vaporization point lower than the boiling point of the highest boiling of the reactants, with the pressure in the reaction zone being between 0,05 bar(a) and 3 bar(a), so that the binary mixture is in the vapour phase, and with a molar excess of the carboxylic acid being present in the binary mixture.

7. A process according to Claim 6, wherein the molar ratio of the carboxylic acid to the alcohol is about 1,5:1,0.

8. A process according to Claim 6 or Claim 7, wherein the vaporized feedstock includes up to 8% by mass of vaporized water.

9. A process according to any one of Claims 6 to 8 inclusive, wherein the vaporized feedstock includes up to 20% by mass of the carboxylic acid ester(s) produced in the process, as a recycle component.

10. A process according to any one of Claims 1 to 9 inclusive, wherein the second reactant comprises a Fischer-Tropsch derived alcohol mixture.

11. A process according to any one of Claims 1 to 10 inclusive, wherein the first reactant comprises Fischer-Tropsch derived acetic acid or propionic acid.

12. A process according to any one of Claims 1 to 10 inclusive, wherein the first reactant comprises a Fischer-Tropsch derived acetic acid/propionic acid mixture.

13. A process according to Claim 1, wherein a molar excess of the carboxylic acid is used.

14. A process according to Claim 13, wherein the molar ratio of the carboxylic acid to the alcohol is about 1,5:1,0.

15. A process according to Claim 1, wherein the first reactant is a Fischer-Tropsch derived alcohol mixture.

**16.** A process according to Claim 1, wherein the second reactant is a Fischer-Tropsch derived carboxylic acid mixture.

**Patentansprüche**

**1.** Verfahren zur Herstellung eines Carbonsäureesters, wobei das Verfahren die Reaktion eines ersten Reaktanten, der zumindest eine Carbonsäure umfaßt, mit einem zweiten Reaktanten, der zumindest einen Alkohol umfaßt, umfaßt, wobei die sich die Carbonsäure und/oder der Alkohol von Fischer-Tropsch ableiten und die Reaktion bei erhöhter Temperatur und bei einem solchen Druck ausgeführt wird, daß sich die Reaktanten in der Gasphase befinden, wobei die Reaktanten weiterhin in Gegenwart eines Kationenaustauschharzes als ein temperaturstabiler, fester, saurer Katalysator ausgeführt wird, um dadurch zumindest einen Carbonsäureester herzustellen.

**2.** Verfahren nach Anspruch 1, wobei das Kationenaustauschharz ein sulfoniertes Polystyren- oder Polydivinylbenzenharz, ein sulfoniertes Polysiloxanharz oder ein sulfoniertes perfluoriertes Harz ist.

**3.** Verfahren nach Anspruch 2, wobei das sulfonierte Polystyrenharz als Katalysator verwendet wird und die erhöhte Temperatur, bei der die Reaktion stattfindet, 150 °C nicht übersteigt.

**4.** Verfahren nach Anspruch 2, wobei das sulfonierte Polysiloxanharz als Katalysator verwendet wird und die erhöhte Temperatur, bei der die Reaktion stattfindet, 230 °C nicht übersteigt.

**5.** Verfahren nach Anspruch 2, wobei das sulfonierte perfluorierte Harz als Katalysator verwendet wird und die erhöhte Temperatur, bei der die Reaktion stattfindet, 200 °C nicht übersteigt.

**6.** Verfahren nach einem der Ansprüche 1 bis einschließlich 5, bei dem die Reaktion des ersten und zweiten Reaktanten in einer Reaktionszone ausgeführt wird, die ein Festbett des Katalysators in Partikelform enthält, durch die die Reaktanten strömen, wobei die Reaktanten in die Reaktionszone als verdampftes Einsatzprodukt eingeführt werden, das ein binäres Gemisch der Reaktanten umfaßt, das einen geringeren Siede- und Verdampfungspunkt hat als der Siedepunkt des am höchsten siedenden Reaktanten, wobei der Druck in der Reaktionszone zwischen 0,05 bar und 3 bar liegt, so daß sich das binäre Gemisch in der Gasphase befindet und wobei ein molarer Überschuß der Carbonsäure in dem binären Gemisch vorliegt.

**7.** Verfahren nach Anspruch 6, wobei das Molverhältnis der Carbonsäure zu dem Alkohol etwa 1,5 : 1,0 ist.

**8.** Verfahren nach Anspruch 6 oder Anspruch 7, wobei das verdampfte Einsatzprodukt bis zu 8 Masseprozent verdampftes Wasser umfaßt.

**9.** Verfahren nach einem der Ansprüche 6 bis einschließlich 8, wobei das verdampfte Einsatzprodukt bis zu 20 Masseprozent Carbonsäureester, der(die) in dem Verfahren hergestellt wurde(n), als rückgeführte Komponente umfaßt.

**10.** Verfahren nach einem der Ansprüche 1 bis einschließlich 9, wobei der zweite Reaktant ein von Fischer-Tropsch abgeleitetes Alkoholgemisch umfaßt.

**11.** Verfahren nach einem der Ansprüche 1 bis einschließlich 10, wobei der erste Reaktant eine von Fischer-Tropsch abgeleitete Essigsäure oder Propionsäure umfaßt.

**12.** Verfahren nach einem der Ansprüche 1 bis einschließlich 10, wobei der erste Reaktant ein von Fischer-Tropsch abgeleitetes Essigsäure/Propionsäure-Gemisch umfaßt.

**13.** Verfahren nach Anspruch 1, wobei ein molarer Überschuß der Carbonsäure verwendet wird.

**14.** Verfahren nach Anspruch 13, wobei das Molverhältnis der Carbonsäure zu dem Alkohol etwa 1,5 : 1,0 ist.

**15.** Verfahren nach Anspruch 1, wobei der erste Reaktant ein von Fischer-Tropsch abgeleitetes Alkoholgemisch ist.

**16.** Verfahren nach Anspruch 1, wobei der zweite Reaktant ein von Fischer-Tropsch abgeleitetes Carbonsäuregemisch ist.

**EP 0 944 572 B1**

## Revendications

1. Procédé de production d'un ester d'acide carboxylique, ledit procédé comprenant la mise en réaction d'un premier réactif comprenant au moins un acide carboxylique avec un deuxième réactif comprenant au moins un alcool, l'acide carboxylique et/ou l'alcool étant dérivé de Fischer-Tropsch et la réaction étant réalisée à une température élevée et à une pression telle que les réactifs se trouvent en phase gazeuse, la réaction étant, en outre, réalisée en présence d'une résine échangeuse d'ions cationiques comme catalyseur acide solide stable en température, pour produire ainsi au moins un ester d'acide carboxylique.

2. Procédé selon la revendication 1, dans lequel la résine échangeuse d'ions cationiques est une résine sulfonée de polystyrène ou de polydivinylbenzène, une résine sulfonée de polysiloxane ou une résine sulfonée perfluorée.

3. Procédé selon la revendication 2, dans lequel la résine sulfonée de polystyrène est utilisée comme catalyseur et dans lequel la température élevée à laquelle se produit la réaction ne dépasse pas 150 °C.

4. Procédé selon la revendication 2, dans lequel la résine sulfonée de polysiloxane est utilisée comme catalyseur et dans lequel la température élevée à laquelle se produit la réaction ne dépasse pas 230 °C.

5. Procédé selon la revendication 2, dans lequel la résine sulfonée perfluorée est utilisée comme catalyseur et dans lequel la température élevée à laquelle se produit la réaction ne dépasse pas 200 °C.

6. Procédé selon l'une quelconque des revendications 1 à 5 comprise, dans lequel la réaction du premier et du deuxième réactif est réalisée dans une zone de réaction comprenant un lit fixe du catalyseur sous forme particulaire à travers lequel passent les réactifs, les réactifs étant alimentés dans la zone de réaction sous forme d'un substrat vaporisé comprenant un mélange binaire des réactifs dont le point d'ébullition ou de vaporisation est inférieur au point d'ébullition du réactif ayant le point d'ébullition le plus élevé, la pression dans la zone de réaction étant comprise entre 0,05 bar(a) et 3 bars(a) de manière à ce que le mélange binaire se trouve en phase vapeur et avec un excédent molaire de l'acide carboxylique présent dans le mélange binaire.

7. Procédé selon la revendication 6, dans lequel le rapport molaire de l'acide carboxylique à l'alcool est d'environ 1,5 / 1,0.

8. Procédé selon la revendication 6 ou la revendication 7, dans lequel le substrat vaporisé comprend jusqu'à 8 % en masse d'eau vaporisée.

9. Procédé selon l'une quelconque des revendications 6 à 8 comprise, dans lequel le substrat vaporisé comprend jusqu'à 20 % en masse de(s) l'ester(s) d'acide carboxylique produit(s) par le procédé, comme constituant de recyclage.

10. Procédé selon l'une quelconque des revendications 1 à 9 comprise, dans lequel le deuxième réactif comprend un mélange d'alcool dérivé de Fischer-Tropsch.

11. Procédé selon l'une quelconque des revendications 1 à 10 comprise, dans lequel le premier réactif comprend de l'acide acétique ou de l'acide propionique dérivé de Fischer-Tropsch.

12. Procédé selon l'une quelconque des revendications 1 à 10 comprise, dans lequel le premier réactif comprend un mélange d'acide acétique et d'acide propionique dérivé de Fischer-Tropsch.

13. Procédé selon la revendication 1, dans lequel on utilise un excédent molaire de l'acide carboxylique.

14. Procédé selon la revendication 13, dans lequel le rapport molaire de l'acide carboxylique à l'alcool est d'environ 1,5 / 1,0.

15. Procédé selon la revendication 1, dans lequel le premier réactif est un mélange d'alcool dérivé de Fischer-Tropsch.

16. Procédé selon la revendication 1, dans lequel le deuxième réactif est un mélange d'acide carboxylique dérivé de Fischer-Tropsch.

FIG 1

FIG 2